Europäisches Patentamt

European Patent Office

Office européen des brevets

(19)

(11) Numéro de publication : **0 434 506 B1**

## (12) FASCICULE DE BREVET EUROPEEN

(45) Date de publication du fascicule du brevet :
**17.03.93 Bulletin 93/11**

(51) Int. Cl.⁵ : **G01T 1/161, A61B 6/03**

(21) Numéro de dépôt : **90403550.8**

(22) Date de dépôt : **12.12.90**

(54) **Procédé de calibration de la chaîne de mesure d'un appareil à rayons X.**

(30) Priorité : **14.12.89 FR 8916568**

(43) Date de publication de la demande :
**26.06.91 Bulletin 91/26**

(45) Mention de la délivrance du brevet :
**17.03.93 Bulletin 93/11**

(84) Etats contractants désignés :
**DE NL**

(56) Documents cités :
**EP-A- 0 321 289**
**US-A- 4 068 306**
**US-A- 4 686 695**
**MEDICAL PHYSICS, vol. 13, no. 3, mai/juin 1986, pages 334-339, Am. Assoc. Phys. Med., New York, US ; W.A. KALENDER et al.: "Evolution of a prototype dual-energy computed tomographic apparatus. I. Phantom studies"**

(73) Titulaire : **GENERAL ELECTRIC CGR S.A.**
**100, rue Camille-Desmoulins**
**F-92130 Issy les Moulineaux (FR)**

(72) Inventeur : **Picard, Catherine**
**Cabinet Ballot-Schmit, 7, rue Le Sueur**
**F-75116 Paris (FR)**
Inventeur : **Rougée, Anne**
**Cabinet Ballot-Schmit, 7, rue Le Sueur**
**F-75116 Paris (FR)**
Inventeur : **Saint-Félix, Didier**
**Cabinet Ballot-Schmit, 7, rue Le Sueur**
**F-75116 Paris (FR)**
Inventeur : **Trousset, Yves**
**Cabinet Ballot-Schmit, 7, rue Le Sueur**
**F-75116 Paris (FR)**

(74) Mandataire : **Schmit, Christian Norbert Marie et al**
**Cabinet Ballot-Schmit 7, rue Le Sueur**
**F-75116 Paris (FR)**

Jouve, 18, rue Saint-Denis, 75001 PARIS

## Description

La présente invention a pour objet un procédé de calibration de la chaîne de mesure d'un appareil à rayons X. Elle trouve plus particulièrement son application dans le domaine médical où les appareils à calibrer sont principalement des appareils à rayons X destinés à mettre en oeuvre des expérimentations à deux énergies. Elle peut néanmoins trouver son application dans d'autres domaines.

Dans un couple d'expérimentations médicales à deux énergies, on soumet au cours d'une première acquisition le corps d'un patient à examiner à une première irradiation alors que le tube à rayons X émet des rayons X dont l'énergie appartient à une première gamme. On acquiert alors une première image. Puis on réitère l'expérimentation pour la deuxième gamme et on acquiert une deuxième image. A partir des deux images acquises on crée des autres images, dans lesquelles la valeur des éléments d'image est fonction d'une composition énergétique des résultats de mesure d'absorption radiologique contenus dans les deux images acquises. Le mode de composition énergétique des images permet, dans une application typiquement médicale, d'extraire en particulier une image à deux dimensions représentative des structures osseuses du corps d'un patient à partir des images acquises.

Les principes de base de la combinaison énergétique sont connus sous le nom de "décomposition par double énergie". Cette décomposition permet, lorsque l'on est en présence d'un objet composé de deux matériaux différents (par exemple ici l'os et les tissus mous) de calculer à partir de deux images en projections de cet objet acquises avec des rayons X d'énergies différentes, une image en projection correspondant à la contribution d'un seul des matériaux composant l'objet, en l'occurrence les os.

Ces principes de base peuvent être trouvés dans les références suivantes: "A Method for Selective Tissue and Bone Visualisation Using Dual Energy Scanned Projection Radiography", W.R. BRODY, G. BUTT, A. HALL and A. MACOVSKI, MED. PHYS., Vol. 8, Numéro 3, Mai/Juin 1981, pages 353-357; et dans "Generalised Image Combinations in Dual k Vp Digital Radiography" L.A. LEHMANN & AI., MED. PHYS., Vol 8, N° 5, sept-oct 1981, pages 659-667. Ces principes reposent sur l'hypothèse fondamentale suivante: pour l'ensemble des corps existants, l'espace des coefficients d'atténuation des rayons X, considéré comme fonction de l'énergie, est un espace à deux dimensions.

Cette hypothèse est physiquement justifiée par le fait que l'absorption des rayons X résulte, aux énergies qui intéressent le domaine médical, de deux phénomènes seulement. Un premier phénomène est l'effet Compton, un deuxième phénomène est l'effet photoélectrique. Plus précisément, le comportement par rapport aux rayons X d'un matériau M donné est entièrement caractérisé par deux grandeurs scalaires. Une première grandeur scalaire est le coefficient d'absorption photoélectrique, noté $a_p$, et la deuxième est le coefficient d'absorption Compton, noté $a_c$. L'hypothèse fondamentale revient à supposer qu'il existe alors deux fonctions de l'énergie $f_p(E)$ et $f_c(E)$ indépendantes des matériaux considérés. Ces deux fonctions sont telles que, quel que soit le matériau M, et quelle que soit l'énergie E, un coefficient d'atténuation massique $\mu_M(E)$ quelconque peut s'écrire sous la forme:

$$\text{I} \qquad \mu_M(E)/\delta_M \; = \; a_p(M) \cdot f_p(E) \; + \; a_c(M) \cdot f_c(E)$$

où $a_p(M)$ et $a_c(M)$ sont fonction du numéro atomique Z et du numéro de masse A du matériau M considéré; $\delta M$ est la densité du matériau M. Ceci revient à dire que les fonctions $f_p(E)$ et $f_c(E)$ forment une base de l'espace des coefficients d'atténuation.

En pratique, on préfère travailler dans une autre base dont les vecteurs de base sont deux autres fonctions de l'énergie E, linéairement indépendantes. Ces fonctions de base sont les coefficients d'atténuation de deux matériaux différents: par exemple d'une part de l'Aluminium Al et, d'autre part, un Polyméthacrylate Po. Dans l'un de ces matériaux, l'absorption photoélectrique est prédominante, par contre, dans l'autre, c'est l'absorption Compton. On choisit l'Aluminium et le Polyméthacrylate parce que l'Aluminium a des propriétés d'absorption voisines de celle de l'os et parce que le Polyméthacrylate a des propriétés d'absorption voisines de celle des tissus mous qui entourent les os. Ceci présente l'intérêt de diminuer les erreurs dues à un certain nombre d'approximations intervenant dans la méthode. Il est possible bien entendu de choisir un autre couple de matériaux au départ. En particulier, le Polyméthacrylate pourrait être remplacé avantageusement par de l'eau. Le Polyméthacrylate a été choisi pour une plus grande simplicité d'utilisation dans une phase de calibration expliquée ci-après. Dans tout ce qui suit, pour des facilités de compréhension, on fait apparaître un choix (Po, Al). Mais le raisonnement théorique ne dépend absolument pas de ce choix et pour d'autres applications que médicales d'autres matériaux peuvent être retenus.

Le changement de base annoncé s'obtient facilement en écrivant la relation I pour les matériaux Al et Po :

$$\mu_{Al}(E)/\delta_{Al} = a_p(Al).f_p(E) + a_c(Al).f_c(E)$$

II

$$\mu_{Po}(E)/\delta_{Po} = a_p(Po).f_p(E) + a_c(Po).f_c(E)$$

puis en inversant le système linéaire II afin d'obtenir l'expression de $f_p(E)$ et $f_c(E)$ en fonction de $\mu Al/\delta Al$ et de $\mu_{Po}/\delta_{Po}$ et enfin en remplaçant dans l'équation I écrite pour un matériau M, $f_p(E)$ et $f_c(E)$ par leurs expressions.

De ces deux expressions II, on peut donc déduire l'expression des fonctions $f_p(E)$ et $f_c(E)$. Celles-ci sont elles-mêmes données maintenant en fonction des coefficients d'absorption globaux $\mu$ de l'Aluminium et du Polyméthacrylate. Si on remplace alors, dans la première expression de $\mu_M(E)$, ces fonctions $f_p(E)$ et $f_c(E)$ par leurs expressions en fonction de $\mu_{Al}$ et $\mu_{Po}$, on obtient une expression de la forme suivante :

III $\qquad \mu_M(E)/\delta_M = K(M)\cdot\mu_{Al}(E)/\delta_{Al} + H(M)\cdot\mu_{Po}(E)/\delta_{Po}$

ou encore:

IV $\qquad \mu_M(E) = K(M)\cdot\mu_{Al}(E)\cdot\delta_M/\delta_{Al} + H(M)\cdot\mu_{Po}(E)\cdot\delta_M/\delta_{Po}$

$K(M)$ et $H(M)$ sont des grandeurs scalaires dépendant des numéros Z et A du matériau M et des matériaux Al et Po.

On utilise par ailleurs un "plan des matériaux" défini de la manière suivante. Considérons un rayon X traversant un objet composé éventuellement de plusieurs matériaux. En chaque point $(x,y,z)$ de l'objet situé sur le trajet du rayon X, trajet que nous appellerons chemin C, on définit la fonction $\mu(x,y,z,E)$ qui est la valeur du coefficient d'atténuation du matériau présent dans l'objet au point $(x,y,z)$ pour l'énergie E. Localement, chaque matériau a un coefficient d'atténuation vérifiant la relation IV, c'est-à-dire s'exprimant comme une combinaison linéaire de $\mu_{Al}$ et $\mu_{Po}$. Par conséquent, l'intégrale des coefficients d'atténuation le long du chemin C vérifie la relation suivante, quelle que soit l'énergie E :

$$V \qquad \int_C \mu(x,y,z,E)\,ds = t_{Po}.\mu_{Po}(E) + t_{Al}.\mu_{Al}(E)$$

$$\text{avec} \qquad t_{Po} = (1/\delta_{Po}).\int_C H(x,y,z)\,\delta_M(x,y,z)\,ds$$

$$\text{et} \qquad t_{Al} = (1/\delta_{Al}).\int_C K(x,y,z)\,\delta_M(x,y,z)\,ds$$

Dans ces expressions, $t_{Al}$ et $t_{Po}$ s'expriment en unité de longueur, et sont appelées épaisseurs équivalentes en Aluminium et Polyméthacrylate.

On définit alors un espace affine bidimensionnel, que l'on appelle "plan des matériaux", de repère orthonormé (O,i,j). Un objet, traversé par un rayon X d'énergie quelconque a, dans cet espace affine, un point associé et un seul, M, de coordonnées $(t_{Po}, t_{Al})$. Un objet en Polyméthacrylate a un point associé porté par l'axe Po colinéaire à i, un objet en Aluminium a un point associé porté par l'axe Al colinéaire à j.

On va voir que les coordonnées polaires $(t_m, \Theta_M)$ d'un vecteur OM sont particulièrement bien adaptées à la représentation d'un matériau homogène. De l'expression V on peut déduire, pour un matériau homogène de longueur L et quelle que soit l'énergie E :

VI $\qquad \mu_M(E)\cdot L = t_{Po}\cdot\mu_{Po}(E) + t_{Al}\cdot\mu_{Al}(E)$

avec $\qquad t_{Po} = H(M)\cdot\delta_M\cdot L/\delta_{Po}$

et $\qquad t_{Al} = K(M)\cdot\delta_M\cdot L/\delta_{Al}$

On déduit deux conséquences à cette formulation, dans le plan des matériaux :

- d'une part, un matériau donné est caractérisé par un rapport pour ce matériau entre $t_{Al}$ et $t_{Po}$. Ce rapport est invariant par changement d'épaisseur ou même changement de densité du matériau. Ceci signifie que l'angle $\Theta_M$, rapporté à l'axe Po de la demi-droite OM qui porte le point M est caractéristique du matériau. Pour l'os, on parlera ainsi de l'angle caractéristique de l'os, soit $\Theta_{os}$. On a la relation :

VI bis $\qquad tg(\Theta_M) = t_{Al}/t_{Po} = \{K(M)/H(M)\}\cdot\{\delta_{Al}/\delta_{Po}\}$

- d'autre part, la norme du vecteur OM, qu'on appelle épaisseur équivalente $t_M$, est proportionnelle au pro-

EP 0 434 506 B1

duit densité-épaisseur $\delta_M.L$ du matériau M :

$$\text{VI ter} \quad t_M = |OM| = t_{PO}.\cos\Theta_M + t_{Al}.\sin\Theta_M$$

$$= \{H(M).\cos\Theta_M / \delta_{PO} + K(M).\sin\Theta_M / \delta_{Al}\}.\delta_M.L$$

Si le matériau M n'a pas une densité constante mais que cette densité est variable spatialement (c'est le cas de l'os, qui peut être plus ou moins compact), alors l'épaisseur équivalente est proportionnelle cette fois-ci à l'intégrale, le long du chemin C, de la densité du matériau considéré. On a en effet alors, d'après l'expression de $t_{Po}$ et $t_{Al}$ dans V :

$$\text{VII} \quad t_{PO} = \int_C H(M)/\delta_{PO}. \ \delta\{x,y,z\}ds$$

$$\text{et} \quad t_{Al} = \int_C K(M)/\delta_{Al}. \ \delta(x,y,z)ds$$

L'angle $\Theta_M$ est encore défini par le rapport $t_{Al}/t_{Po}$, rapport indépendant de la densité :

$$\text{VII bis} \quad tg(\Theta_M) = t_{Al}/t_{Po} = \{K(M)/H(M)\}\cdot\{\delta_{Al}/\delta_{Po}\}$$

et l'épaisseur équivalente, soit $|OM|$, vaut :

$$\text{VII ter}$$

$$t_M = |OM| = \{H(M).\cos\Theta_M / \delta_{PO} + K(M).\sin\Theta_M / \delta_{Al}\} \int_C \delta(x,y,z)ds$$

Dans le cas qui intéresse la médecine, l'objet traversé par le rayon X est composé de plusieurs matériaux, pour le moins des os et des tissus mous, d'angles caractéristiques respectivement $\Theta_{os}$ et $\Theta_{ti}$.

On accepte ici de considérer que tous les tissus mous sont équivalents. Ceci n'est pas vrai dans la mesure où, par exemple, les muscles et les graisses n'ont pas les mêmes propriétés d'absorption, c'est-à-dire pas les mêmes coefficients $a_p$ et $a_c$. Cependant, compte tenu de ce que leurs angles sont sensiblement comparables, et en tous cas fort éloignés de celui de l'os, cette approximation se justifie.

On peut, dans le plan des matériaux, représenter le vecteur OM correspondant au corps, comme étant la somme des deux vecteurs $OM_{os}$ et $OM_{ti}$, correspondant chacun aux contributions de l'os et des tissus mous. Chacun de ces deux vecteurs fait un angle de $\Theta_{os}$ et $\Theta_{ti}$ respectivement avec (O,i), et a une longueur de respectivement $t_{os}$ et $t_{ti}$. Ces longueurs sont proportionnelles au produit épaisseur-densité d'os et de tissu mou traversés.

La connaissance de $\Theta_{os}$ et $\Theta_{ti}$ permet d'effectuer un changement de repère dans le plan des matériaux, pour passer du repère (O,i,j) au repère (O,u,v) avec u vecteur unitaire porté par l'axe des tissus mous et v vecteur unitaire porté par l'axe des os. Ainsi, on peut exprimer les "longueurs" équivalentes os et tissu mou en fonction des "longueurs équivalentes Aluminium et Polyméthacrylate" :

$$\text{VIII} \quad t_{os} = \{-t_{Po}\sin\Theta_{ti} + t_{Al}\cos\Theta_{ti}\}/\sin(\Theta_{os}-\Theta_{ti})$$

$$t_{ti} = \{ t_{Po}\sin\Theta_{os} - t_{Al}\cos\Theta_{os}\}/\sin(\Theta_{os}-\Theta_{ti})$$

4

On va voir maintenant que l'on peut déterminer, pour un corps donné, les "épaisseurs équivalentes" $t_{Po}$ et $t_{Al}$ à partir des mesures d'atténuation de rayonnement faites à deux énergies d'émission du rayonnement X différentes, rayonnements auxquels on soumet ce corps.

Dans le cas d'un rayonnement monochromatique d'énergie E, on peut écrire la relation suivante :

$$IX \qquad I = I_0 . e^{- \int \mu_M(x,y,z,E).ds}$$

Où I est l'intensité de rayonnement mesurée sur un détecteur après traversée de l'objet, et $I_0$ est l'intensité qui serait mesurée sur le même détecteur si le rayonnement n'avait pas traversé l'objet ($I_0$ est appelé dans la suite intensité à feu nu).

L'équation IX se transforme aisément en

$$IX\ bis \qquad Ln(I_0/I) = - \int_C \mu_M(x,y,z,E).ds$$

En appliquant la relation V, on obtient alors :

$$X \qquad Ln(I_0/I) = t_{Po}.\mu_{Po}(E) + t_{Al}.\mu_{Al}(E)$$

Dans la suite, on pose U ou V = $Ln(I_0/I)$ selon que l'énergie de rayonnement utilisée est l'une où l'autre des deux énergies E1 et E2. L'équation X écrite pour chacune des deux énergies permet d'obtenir le système :

$$XI \qquad \begin{aligned} U &= t_{Po}.\mu_{Po}(E_1) + t_{Al}.\mu_{Al}(E_1) \\ V &= t_{Po}.\mu_{Po}(E_2) + t_{Al}.\mu_{Al}(E_2) \end{aligned}$$

système qui s'inverse aisément pour obtenir $t_{Po}$ et $t_{Al}$ en fonction des mesures $U_1$ et $U_2$ :

$$XII \qquad \begin{aligned} t_{Po} &= c_{11}.U + c_{12}.V \\ t_{Al} &= c_{21}.U + c_{22}.V \end{aligned}$$

Les coefficients $c_{11}$, $c_{12}$, $c_{21}$, $c_{22}$ ne dépendent que des coefficients d'absorption de l'Aluminium et du Polyméthacrylate aux énergies $E_1$ et $E_2$.

Dans le cas réel rencontré lors d'un examen radiologique, le spectre d'émission des rayons X n'est pas monochromatique. Par exemple, lorsque le tube à rayons X est alimenté en haute tension à 50 kV, les énergies $E_1$ des rayons X émis sont réparties entre environ 10 et 50 KeV. Lorsque le tube est alimenté à 100 kV, les énergies $E_2$ des rayons X sont réparties entre 40 et 100 KeV.

L'absorption d'un rayonnement X de spectre énergétique S(E) au travers d'un corps s'exprime alors par l'équation suivante XIII, dont l'équation IX est un cas particulier.

$$XIII \qquad I = I_0 . \int_E S(E).e^{\int_C -\mu_M(x,y,z,E).ds}\ dE$$

On n'a plus alors, comme on l'avait en IX bis, égalité entre la mesure U ($U = Ln(I_0/I)$) et l'intégrale des coefficients d'atténuation le long du chemin C.

Si l'on veut appliquer la méthode de décomposition par double énergie expliquée plus haut, il est par conséquent nécessaire d'effectuer un certain nombre d'adaptations, aussi bien de la physique à la méthode que de la méthode à la physique.

La première adaptation est de diminuer, dans la mesure du possible, de manière physique l'aspect non monochromatique du spectre. Pour modifier le spectre des rayons X en vue de le rendre plus proche d'un spectre monochromatique, on effectue généralement une filtration du rayonnement par une épaisseur de matériau (métallique par exemple) qui absorbe plus les faibles énergies (rayons X mous) que les énergies élevées. Dans chacun des spectres, on diminuera donc la contribution de la partie gauche du spectre. Ceci présente également l'intérêt de diminuer, et même de rendre pratiquement vide, la région d'intersection des deux spectres correspondant aux deux tensions d'alimentation du tube à rayons X. Ainsi, aucune des énergies du spectre $E_1$ n'est égale à une énergie du spectre $E_2$.

L'adaptation de la méthode à la nature spectrale du rayonnement passe par une généralisation des équations linéaires XII en deux développements de Taylor à l'ordre p (on prend pour simplifier p = 3), de manière à tenir compte de l'inégalité mise en évidence à la suite de XIII. Cette généralisation devient:

$$t_{Po} = c_{11} + c_{12}U + c_{13}V + c_{14}U^2 + c_{15}V^2 + c_{16}UV + c_{17}U^3 + c_{18}V^3$$

XIV

$$t_{Al} = c_{21} + c_{22}U + c_{23}V + c_{24}U^2 + c_{25}V^2 + c_{26}UV + c_{27}U^3 + c_{28}V^3$$

Dans ce système, U désigne la mesure effectuée pour un rayonnement de spectre énergétique S1(E), dit rayonnement "basse énergie", et V désigne la mesure effectuée pour un rayonnement de spectre énergétique S2(E), dit rayonnement "haute énergie". Ici encore, les coefficients $c_{ij}$ ne dépendent que des caractéristiques des matériaux de base Al et Po.

Les coefficients $c_{ij}$ sont de préférence estimés, plutôt que déterminés de manière théorique, au cours d'une phase de calibration, expliquée ci-après.

L'appareil à rayons X comporte un détecteur à deux dimensions. On soumet le détecteur de cet appareil à un rayonnement X "basse énergie", en l'absence d'objet entre la source et le détecteur, ce qui permet la mesure de l'intensité à feu nu $I_{01}$. On mesure de la même façon $I_{02}$, intensité à feu nu pour un rayonnement X "haute énergie".

On soumet ensuite une épaisseur connue d'Aluminium, $t_{Al}$ superposée à une épaisseur connue de Polyméthacrylate, $t_{Po}$, au même rayonnement "basse énergie", ce qui permet la mesure de l'intensité $I_1$, puis au même rayonnement "haute énergie", ce qui permet la mesure de l'intensité $I_2$. On en déduit U et V

On renouvelle l'opération de mesure de $I_1$ et $I_2$ pour un certain nombre de couples $(t_{Po}, t_{Al})$, soient N couples d'épaisseurs empilées de ces matériaux. A chaque couple correspond un point M dans le "plan des matériaux". On appelle alors "couples différents" deux couples de points correspondants M1 et M2, tels que les vecteurs OM1 et OM2 ne sont pas colinéaires.

Pour chaque couple, on applique les équations XIV. On obtient alors deux systèmes de N équations linéaires s'écrivant sous une forme matricielle XV où les inconnues sont les coefficients $c_{ij}$, la matrice du système étant appelée "matrice des mesures" :

XV

$$
\begin{vmatrix} t_{Po1} \\ t_{Po2} \\ \\ --- \\ \\ t_{PoN} \end{vmatrix}
=
\begin{vmatrix} 1 & U_1 & V_1 & U_1^2 & V_1^2 & U_1V_1 & U_1^3 & V_1^3 \\ 1 & U_2 & V_2 & U_2^2 & V_2^2 & U_2V_2 & U_2^3 & V_2^3 \\ \\ - & -- & -- & --- & --- & ---- & --- & --- \\ \\ 1 & U_N & V_N & U_N^2 & V_N^2 & U_NV_N & U_N^3 & V_N^3 \end{vmatrix}
\begin{vmatrix} c_{11} \\ c_{12} \\ \\ --- \\ \\ c_{1N} \end{vmatrix}
$$

EP 0 434 506 B1

de même pour l'Aluminium :

$$
\begin{vmatrix} t_{Al1} \\ t_{Al2} \\ \cdot \\ --- \\ \\ t_{AlN} \end{vmatrix} = \begin{vmatrix} \text{MATRICE DES MESURES} \end{vmatrix} \begin{vmatrix} c_{21} \\ c_{22} \\ \\ --- \\ \\ c_{2N} \end{vmatrix}
$$

Dans le cas d'un développement de TAYLOR à l'ordre 3, huit couples différents (N=8) suffisent à l'inversion de la matrice des mesures et au calcul des coefficients $c_{ij}$. En pratique, on préfère utiliser dans la phase de calibration beaucoup plus que huit couples différents, et on inverse alors la matrice des mesures au sens des moindres carrés.

Après la phase qui a permis de déterminer les coefficients $c_{ij}$, on pratique les acquisitions "basse énergie" et "haute énergie" pour le corps du patient que l'on veut examiner. On obtient alors deux mesures U et V par élément d'image, ou pixel, pour les images acquises. Les épaisseurs équivalentes de Polyméthacrylate et d'Aluminium sont alors le résultat des équations XIV appliquées à ces valeurs U et V. Les épaisseurs équivalentes Os et Tissus mous sont alors le résultat des équations VIII.

Dans le cas de la radiologie classique (projection 2D de l'objet sur le détecteur 2D), les mesures des intensités I et $I_0$ sont remplacées par les niveaux de gris alloués aux pixels dans l'image 2D correspondant au cellules du multi-détecteur plan qui interceptent le trajet du rayon X. Ainsi, si G est le niveau de gris pour un pixel donné dans l'image avec objet, et si $G_0$ est le niveau de gris pour le même pixel dans l'image à feu nu, on écrit :

$$\text{XVI} \qquad U = Ln(I_0/I) = Ln(G_0/G), \text{ il en est de même pour V.}$$

Les épaisseurs équivalentes pour l'objet à examiner doivent être alors calculées pixel par pixel, à partir des mesures faites pixel par pixel dans les images "basse énergie" et "haute énergie". L'image résultat, dite "image épaisseur équivalente" est formée par l'attribution à chacun de ses pixels d'un niveau de gris égal ou proportionnel à la valeur de l'épaisseur équivalente choisie, c'est à dire calculée pour ce pixel.

On obtient alors, par exemple, une image "épaisseur équivalente d'os", où le niveau de gris n'est pas nul, théoriquement, uniquement pour les pixels correspondant à un rayon ayant traversé de l'os. Pour les pixels correspondant à la projection d'une structure osseuse, le niveau de gris est alors proportionnel au produit densité-épaisseur d'os, ou plus exactement à l'intégrale de la densité osseuse le long du rayon, le coefficient de proportionnalité ne dépendant pas des matériaux (tissus mous) présents autour de l'os.

Dans le calcul de l'image "épaisseur équivalente" décrit plus haut, il est implicite que le système d'acquisition utilisé est un système "parfait", n'introduisant pas de non-uniformités. Comme ce n'est pas la cas en réalité, les non-uniformités dans les mesures qui ne sont pas (ou qui sont imparfaitement) corrigées se traduisent alors par des erreurs dans l'estimation des épaissseurs équivalentes.

Dans l'état de l'art, lors d'une combinaison "double énergie", les coefficients $c_{ij}$ sont estimés globalement. Ces coefficients sont estimés à partir de mesures effectuées sur deux images (une pour chaque énergie) d'une "marche d'escalier" de Polyméthacrylate croisée avec une autre "marche d'escalier" d'Aluminium. On effectue alors les mesures de calibration pour les couples d'épaisseur résultant du croisement de ces "marches d'escalier".

Dans l'état de l'art, on peut alors compenser les non-uniformités du système par une correction des mesures de calibration, préalable au calcul des coefficients $c_{ij}$, suivie d'une correction des mesures concernant le corps du patient, préalable à la combinaison polynomiale. De telles corrections préalables nécessitent la connaissance exacte de la fonction de transfert du système pour chaque point de l'image bidimensionnelle, ce qui est rendu difficile par la dépendance à de nombreux paramètres (énergies, intensités, géométrie, défauts du détecteur....) De plus, même si cette fonction de transfert était bien connue, l'application de ces corrections serait lourde puisque devant s'effectuer sur toutes les mesures.

Dans le cas de la reconstruction 3D sélective à partir d'images combinées par double-énergie, il est nécessaire que les images "épaisseur équivalente" soient porteuses d'une information quantitative. Par exemple, pour une image "épaisseur équivalente d'os", il est nécessaire que le niveau de gris de chaque pixel soit bien proportionnel à l'épaisseur réelle d'os traversée, ou bien à l'intégrale des densités osseuses le long du trajet

7

(le coefficient de proportionnalité étant constant spatialement). Si cette condition n'est pas vérifiée, les données servant à la reconstruction seront dites "non consistantes" et amèneront à la génération d'artefacts de reconstruction.

L'invention a pour objet de remédier à ces inconvénients sans par ailleurs imposer des compensations d'uniformité dans l'élaboration de toutes les images: images acquises, images des matériaux, et images élaborées par combinaison doubles énergie. Pour obtenir alors une information quantitative sur toute l'image, plutôt que de corriger a priori le système, on procède en considérant que chaque cellule du détecteur est un détecteur indépendant, et on estime cellule par cellule l'épaisseur équivalente, c'est-à-dire qu'implicitement, à chaque cellule (x,y) du détecteur correspond un jeu de coefficients $c_{ij}$ (x,y) dépendant de la position de cette cellule. La généralisation que l'on a adoptée en XIV permet de tenir compte, pour chaque cellule, des distorsions de mesure décrites ci-dessus qui se traduisent notamment par une inexactitude de la formule XVI. De cette manière, on intègre, au moment du calcul de combinaison énergétique, la correction de non uniformité dans le calcul des épaisseurs équivalentes. De ce fait aucune correction de compensation ne doit être pratiquée: la compensation d'uniformité est faite, pixel par pixel, quand on calcule les épaisseurs équivalentes avec des coefficients qui la comporte déjà.

L'invention a donc pour objet un procédé de calibration de la chaîne de mesure d'un appareil à rayons X destiné à une acquisition comportant deux phases, chaque phase étant menée avec une énergie différente de rayonnement X, dans lequel

- on émet, au cours de ces deux phases, avec un tube à rayons X de cet appareil, un desdits rayonnements X aux deux énergies en direction d'un corps d'un patient à examiner,
- on acquiert pendant chacune de ces deux phases au moins une image en projection du corps, ces images étant constituées par des collections de résultats de mesure, à l'endroit de cellules d'un détecteur à plusieurs cellules de cet appareil, de l'absorption radiologique de ces rayonnements dans ce corps,
- on traite ces résultats de mesure d'absorption radiologique de chacune de ces phases, en effectuant une combinaison énergétique deux à deux de ces images en projection acquises alors que le rayonnement X est émis avec des énergies différentes,
- ce traitement comportant une calibration,
- cette calibration comportant, préalablement, pour ces deux énergies, des mesures spécifiques de l'absorption radiologique desdits rayonnements au travers d'empilages de deux matériaux couvrant la totalité de la superficie du détecteur
- ces deux matériaux ayant des comportements en absorption radiologique différents,
- ces mesures spécifiques étant destinées à obtenir deux groupes de coefficients décrivant respectivement le comportement en absorption radiologique de chacun de ces deux matériaux en fonction de la valeur de l'énergie, caractérisé en ce que
- cette calibration est effectuée cellule du détecteur par cellule du détecteur, de façon à obtenir deux groupes de coefficients pour chacune des cellules du détecteur.

Dans une mise en oeuvre pratique, le détecteur est un détecteur à deux dimensions du type amplificateur de brillance.

Dans un perfectionnent de l'invention, puisque le calcul des jeux de coefficients $c_{ij}$ (x,y) pour chaque cellule est lourd, puisqu'il conduit à l'inversion d'une matrice des mesures, en plus au sens des moindres carrés, autant de fois qu'il y a de cellules dans le détecteur, on évite d'estimer les coefficients $c_{ij}$ (x,y) en toute cellule du détecteur. Pour toutefois tenir compte de leur variation spatiale, qui est une variation basse fréquence, on estime les coefficients en un certain nombre de cellules réparties sur un maillage carré du détecteur, par exemple 32 cellules par 32 cellules. Puis on estime les coefficients pour les cellules intermédiaires par une méthode d'interpolation, par exemple une méthode d'interpolation bilinéaire.

L'invention sera mieux comprise à la lecture de la description qui suit et à l'examen des figures qui l'accompagnent. Celles-ci ne sont données qu'à titre indicatif et nullement limitatif de l'invention. Les figures montrent:

figure 1: une représentation schématique d'un appareil à rayons X pour lequel on peut mettre en oeuvre le procédé de l'invention;
figure 2: une représentation schématique du mode de combinaison énergétique en vue d'élaborer une autre image;
figure 3: une représentation schématique du caractère non monochromatique des rayonnements X réels;
figure 4: les effets de non uniformité du rendement de détection des détecteurs de rayons X;
figure 5: la représentation d'une des phases du procédé de l'invention;

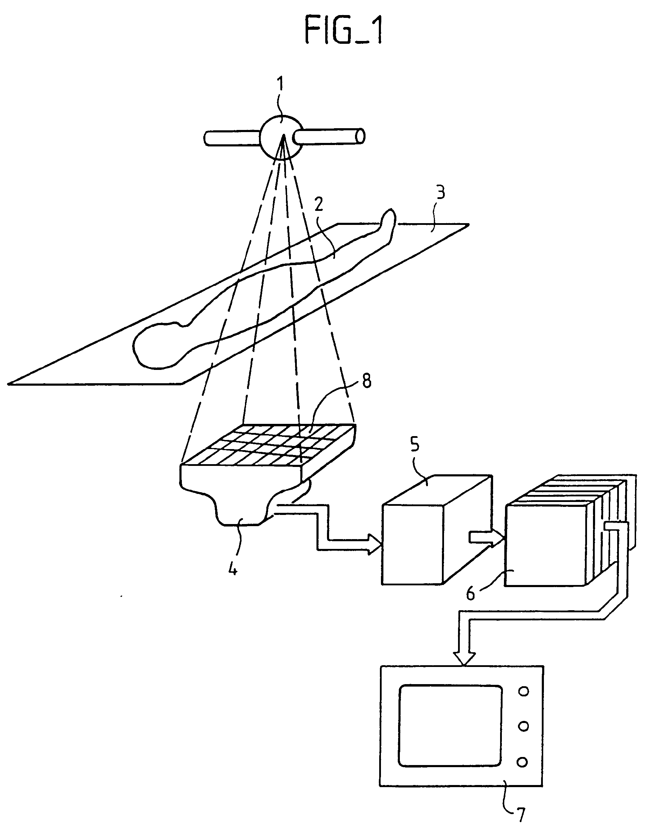

La Figure 1 représente un appareil pour lequel on peut mettre en oeuvre le procédé de l'invention. Dans celui-ci un système d'acquisitions radiologiques 2 D comportant essentiellement un tube à rayons X 1, muni de ses circuits électroniques de commande non spécifiquement représentés, irradie le corps 2 d'un patient placé sur un lit porte-patient 3. A l'opposé du tube 1 par rapport au lit 3 est placé un détecteur 4 dit 2D qui reçoit le rayonnement X après que ce rayonnement ait traversé le corps 2. Dans un exemple, le détecteur 4 peut comporter un intensificateur d'images radiologiques en relation avec une caméra. Le signal délivré par le détecteur 4 est acheminé vers un organe de traitement 5 comportant essentiellement une unité arithmétique et logique et des mémoires. L'organe de traitement 5 peut effectuer la numérisation des images acquises et leur traitement selon l'invention. Ces images sont ensuite stockées dans une mémoire 6. Un moniteur de visualisation 7 ainsi qu'un logiciel de visualisation associé permettent de représenter les structures osseuses comme si on avait disséqué les tissus qui les entourent.

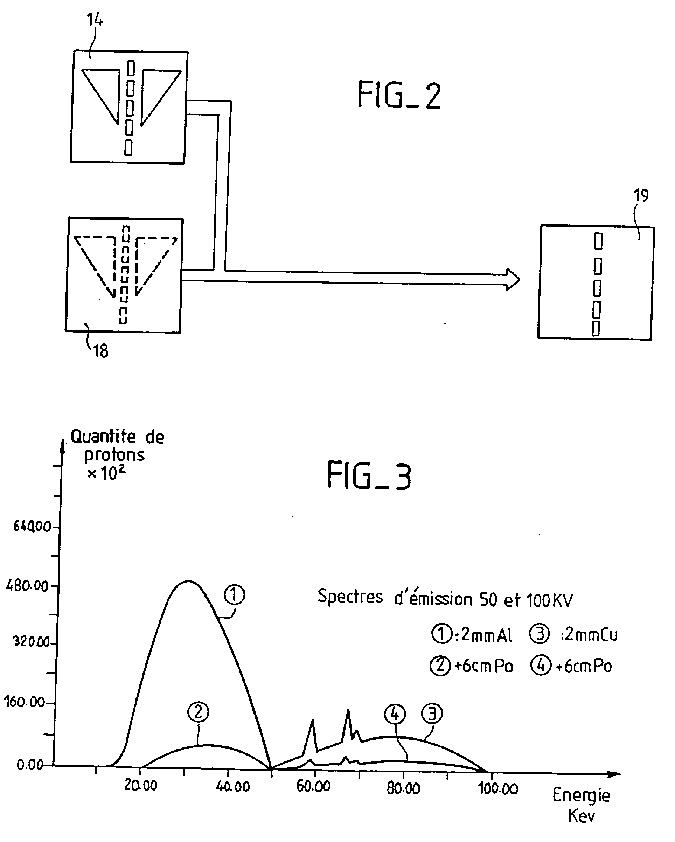

La Figure 2 montre un protocole d'acquisition osseux. Elle montre qu'on peut utiliser une image en projection 14 obtenue alors que le tube a rayons X émet un rayonnement avec un spectre donné, pour la combiner avec une autre projection 18 obtenue alors que le tube à rayons X émet un spectre d'énergie différent. En combinant deux à deux selon un mode de combinaison énergétique évoqué ci-dessus, les informations des pixels des images en projection 14 et 18, on est capable de produire une autre image 19 en projection représentative de la structure osseuse seule.

La figure 3 montre que le spectre d'émission des rayons X n'est pas monochromatique. Par exemple, lorsque le tube à rayons X est alimenté en haute tension à 50 kV, les énergies $E_1$ des rayons X émis sont réparties entre environ 10 et 50 keV. Lorsque le tube est alimenté à 100 kV, les énergies $E_2$ des rayons X sont réparties entre 40 et 100 keV. En figure 3, on a représenté le spectre des rayons X pour les tensions d'alimentation 50 et 100 kV.

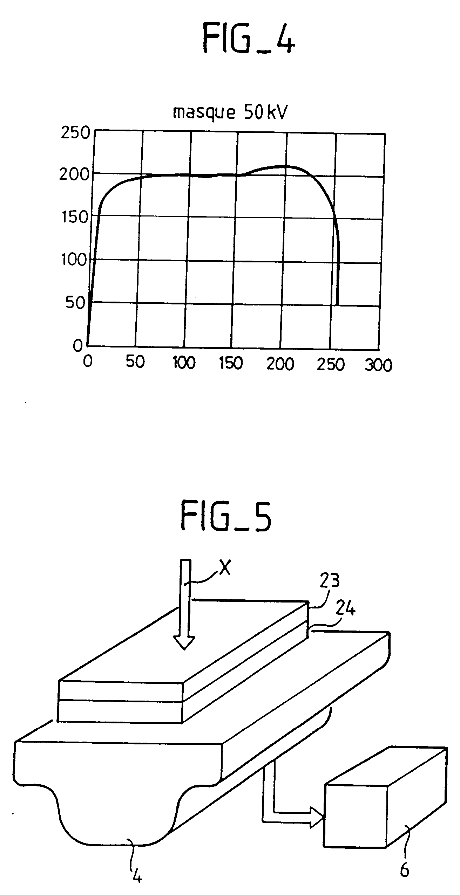

Différents défauts amenés par le système d'acquisition des images radiologiques bidimensionnelles ont amené, dans l'invention, à adapter la phase de calibration de façon à y inclure une correction de ces défauts. Comme le montre la figure 4, d'une part le rendement des cellules détectrices n'est pas uniforme sur toute la surface du détecteur, et n'est pas une fonction linéaire de l'intensité incidente. Il est par exemple plus atténué sur les bords du champ de détection du détecteur. Au milieu du champ ce rendement est également atténué, d'une façon moindre toutefois. D'autre part, le spectre d'émission des rayons X n'est pas isotrope. Enfin, le rayonnement diffusé introduit une différence supplémentaire entre les mesures U et V, et l'intégrale des coefficients d'absorption.

Ainsi, lorsqu'on expose le détecteur à un rayonnement au travers d'un matériau homogène d'épaisseur constante, ou bien lorsqu'on prend une image "à feu nu", le niveau de gris obtenu dans l'image n'est pas uniforme, mais présente des variations "basse fréquence" dont on donne un exemple figure 4. Cet exemple est obtenu avec un détecteur de type intensificateur d'images radiologiques couplé à une caméra. L'épaisseur du dépôt de matériau intensificateur dans ce détecteur ainsi que d'autres facteurs rendent cette variation continue et basse fréquence. Les "distorsions densitométriques" dépendent par ailleurs d'un nombre important de facteurs, tels que les paramètres géométriques de l'acquisition (distance source - détecteur, distance objet-détecteur, rayon de courbure du détecteur...), la nature exacte du spectre d'émission des rayons X dans toutes les directions (puisque celui-ci n'est pas isotrope), le rendement de l'amplificateur d'images, fonction de l'énergie et de l'intensité des rayons incidents...

Le calcul des jeux de coefficients $c_{ij}$ (x,y) pour chaque cellule du détecteur est lourd, puisqu'il conduit à l'inversion d'une matrice des mesures, en plus au sens des moindres carrés, autant de fois qu'il y a de cellules dans le détecteur. Pour éviter d'estimer les coefficients $c_{ij}$ (x,y) en tout point du détecteur, et pour toutefois tenir compte de leur variation spatiale, dans le cas de détecteurs qui présentent une variation basse fréquence et continue, telle que décrite plus haut on a choisi dans cette méthode d'estimer les coefficients en un certain nombre de cellules telles que 8 (figure 1) réparties sur un maillage carré, par exemple de 32 par 32, du détecteur. Puis on estime les coefficients pour les cellules intermédiaires par une méthode d'interpolation, par exemple une interpolation bilinéaire.

Les mesures pour chacune des 32 × 32 cellules sont effectuées à partir d'un jeu d'images, dites mires de calibration, qui sont les images obtenues en interposant entre la source et le détecteur une plaque 23 d'épaisseur constante $t_{Po}$ de Polyméthacrylate superposée à une plaque 24 d'épaisseur constante $t_{Al}$ d'Aluminium, et ceci pour un certain nombre de couples d'épaisseurs ($t_{Po}$, $t_{Al}$). La surface des plaques est plus importante que la surface utile du détecteur pour éviter des effets de bord. Ce jeu de mires contient toutes les informations nécessaires à la calibration.

Le calcul des mesures Ln($G_0$/G) pour chacun des 32 × 32 cellules choisies est de préférence effectué en remplaçant G et $G_0$ par une valeur estimée sur une fenêtre centrée autour du point choisi. Cette valeur est par exemple la valeur moyenne des niveaux de gris sur la fenêtre, ou bien la valeur médiane, ceci en vue de limiter les erreurs apportées par le bruit quantique.

**Revendications**

1. Procédé de calibration de la chaîne de mesure d'un appareil à rayons X destiné à une acquisition comportant deux phases, chaque phase étant menée avec une énergie différente de rayonnement X, dans lequel
   - on émet, au cours de ces deux phases, avec un tube à rayons X de cet appareil, lesdits rayonnements X aux deux énergies en direction d'un corps d'un patient à examiner,
   - on acquiert pendant chacune de ces deux phases au moins une image en projection du corps, ces images acquises étant constituées par des collections de résultats de mesure, à l'endroit de cellules d'un détecteur à plusieurs cellules de cet appareil, de l'absorption radiologique de ces rayonnements dans ce corps,
   - on traite ces résultats de mesure d'absorption radiologique de chacune de ces phases, en effectuant une combinaison énergétique deux à deux des éléments d'image de ces images en projection acquises,
   - ce traitement comportant une calibration,
   - cette calibration comportant, préalablement, pour ces deux énergies, des mesures spécifiques de l'absorption radiologique desdits rayonnements au travers d'empilages de deux matériaux couvrant la totalité de la superficie du détecteur,
   - ces deux matériaux ayant des comportements en absorption radiologique différents,
   - ces mesures spécifiques étant utilisés à obtenir deux groupes de coefficients décrivant respectivement le comportement en absorption radiologique de chacun de ces deux matériaux en fonction de la valeur de l'énergie,
   caractérisé en ce que
   - cette calibration est effectuée cellule du détecteur par cellule du détecteur, de façon à obtenir deux groupes de coefficients pour chacune des cellules du détecteur.

2. Procédé selon la revendication 1, caractérisé en ce que le détecteur est un détecteur à deux dimensions présentant pour ses cellules des variations basses fréquences et continues de ses caractéristiques de détection.

3. Procédé selon la revendication 1 ou la revendication 2, caractérisé en ce que pour effectuer la calibration cellule par cellule
   - on mesure les effets d'absorption à l'endroit des cellules d'un ensemble de cellules choisies,
   - on calcule les coefficients des groupes de coefficients en ces cellules choisies, et
   - on déduit par interpolation bilinéaire les coefficients en des cellules intermédiaires entre ces cellules choisies.

4. Procédé selon l'une quelconque des revendications 1 ou 3, caractérisé en ce que la combinaison énergétique comporte l'élaboration d'au moins une autre image dont la valeur affectée à chaque pixel est fonction d'une épaisseur équivalente en un de ces deux matériaux.

5. Procédé selon l'une quelconque des revendications 1 à 4, caractérisé en ce que pour mesurer les effets d'absorption à l'endroit d'un ensemble de cellules choisies
   - on détermine des fenêtres centrées sur chacune de ces cellules choisies,
   - et on effectue un calcul statistique sur les mesures de détection radiologique en des cellules voisines de la cellule choisie et contenues dans la fenêtre.

**Patentansprüche**

1. Eichverfahren für den Meßkanal eines Röntgenberäts zur Erfassung in zwei Phasen, von denen jede mit einer unterschiedlichen Röntgenstrahlungsenergie betrieben wird, in dem
   - im Verlauf dieser zwei Phasen mit einer Röntgenstrahlröhre dieses Geräts die Röntgenstrahlen mit zwei Energien in Richtung eines zu untersuchenden Körpers eines Patienten emittiert werden,
   - während jeder dieser zwei Phasen wenigstens ein Projektionsbild des Körpers erfaßt wird, wobei diese erfaßten Bilder von Ansammlungen von auf die Zellen eines mehrere Zellen aufweisenden Detektors dieses Geräts bezogenen Meßergebnissen der radiologischen Absorption dieser Strahlungen in diesem Körper gebildet werden,
   - diese Meßergebnisse der radiologischen Absorption einer jeden dieser Phasen bearbeitet werden, indem eine paarweise energetische Kombination der Bildelemente dieser erfaßten Projektionsbilder

EP 0 434 506 B1

bewerkstelligt wird,
- wobei diese Verarbeitung eine Eichung umfaßt,
- wobei diese Eichung zunächst für diese zwei Energien spezifische Messungen der radiologischen Absorption der die Übereinanderschichtungen der die gesamte Oberfläche des Detektors abdeckenden zwei Materialien durchquerenden Strahlungen umfaßt,
- wobei diese zwei Materialien unterschiedliches Verhalten bei radiologischer Absorption zeigen,
- wobei diese spezifischen Messungen dazu verwendet werden, zwei Gruppen von Koeffizienten zu erhalten, die jeweils das Verhalten eines jeden dieser zwei Materialien bei radiologischer Absorption in Abhängigkeit vom Energiewert beschreiben,
        dadurch gekennzeichnet, daß
- diese Eichung von einer Detektorzelle zur nächsten ausgeführt wird, derart, daß für jede der Detektorzellen zwei Gruppen von Koeffizienten erhalten werden.

2. Verfahren gemäß Anspruch 1, dadurch gekennzeichnet, daß der Detektor ein zweidimensionaler Detektor ist, der für seine Zellen niederfrequente und statische Schwankungen seiner Erfassungscharakteristiken aufweist.

3. Verfahren gemäß Anspruch 1 oder Anspruch 2, dadurch gekennzeichnet, daß zur zellenweisen Ausführung der Eichung
- die auf die Zellen einer Gesamtheit von gewählten Zellen bezogenen Absorptionswirkungen bemessen werden,
- die Koeffizienten der Gruppen von Koeffizienten für diese gewählten Zellen berechnet werden und
- durch bilineare Interpolation die Koeffizienten für zwischen diesen gewählten Zellen befindliche Zellen abgeleitet werden.

4. Verfahren gemäß einem der Ansprüche 1 oder 3, dadurch gekennzeichnet, daß die energetische Kombination die Auswertung wenigstens eines weiteren Bildes umfaßt, bei dem der einem jeden Bildelement zugeordnete Wert von einer äquivalenten Dicke in einem dieser zwei Materialien abhängt.

5. Verfahren gemäß einem der Ansprüche 1 bis 4, dadurch gekennzeichnet, daß zur Messung der auf eine Gesamtheit von gewählten Zellen bezogenen Absorptionswirkungen
- Fenster bestimmt werden, die auf jeder dieser gewählten Zellen zentriert sind,
- und eine statistische Rechnung bezüglich der Messungen der radiologischen Erfassung für Zellen ausgeführt wird, die der gewählten Zelle benachbart sind und im Fenster enthalten sind.

## Claims

1. Calibration method for the measuring chain of an X-ray apparatus, intended for an acquisition comprising two phases, each phase being guided with a different X-ray energy, wherein:
- - in the course of these two phases, and with an X-ray tube of this apparatus, the said X-rays with two energies are emitted in the direction of the body of a patient to be examined,
- - during each of these two phases a projection image of the body is acquired, these acquired images consisting of collections of results of measuring, in cells of a multi-cell detector of this apparatus, the radiological absorption of these radiations in this body,
- - these results of measuring the radiological absorption of each of these phases are treated by effecting an energy combination, two by two, of the image elements of these acquired projection images,
- - this treatment includes a calibration,
- - this calibration comprises, in advance, for these two energies, specific measures of the radiological absorption of the said radiations through pilings of two materials covering the whole of the surface of the detector,
- - these two materials have different radiological absoprtion characteristics,
- - these specific measures are used for the purpose of obtaining two groups of coefficients describing, respectively, the radiological absorption behaviour of each of these two materials as a function of the value of the energy,
characterized by the fact that
- - this calibration is effected cell by cell of the detector, in such a way as to obtain two groups of coefficients for each of the cells of the detector.

11

2. Process in accordance with claim 1, characterized by the fact that the detector is a two-dimension detector having, for its cells, low-frequency and continuous variations of its detection characteristics.

3. Process in accordance with claim 1 or claim 2, characterized by the fact that in order to effect the calibration cell by cell the absorption effects are measured in the cells of an assembly of selected cells,
   - - the coefficients of the groups of coefficients in these selected cells are calculated, and
   - - by bi-linear interpolation the coefficients in intermediate cells between these selected cells are deduced.

4. Process in accordance with any one of claims 1 or 3, characterized by the fact that the energy combination process comprises the elaboration of at least one other image of which the value allocated to each pixel is a function of an equivalent thickness in one of these two materials.

5. Process in accordance with any of claims 1 to 4, characterized by the fact that to measure the absorption effects in an assembly of selected cells
   - - windows centered on each of these selected cells are determined,
   - - and a statistical calculation is made of the radiological detection measurements in cells in the vicinity of the selected cell and contained in the window.

# FIG_1

FIG_2

FIG_3

Quantité de protons ×10²

Spectres d'émission 50 et 100KV

①:2mmAl ③ :2mmCu

②+6cmPo ④ +6cmPo

Energie Kev

## FIG_4

masque 50 kV

## FIG_5